(19) Europäisches Patentamt
European Patent Office
Office européen des brevets

(11) Veröffentlichungsnummer: **0 025 552**
**B1**

# (12) EUROPÄISCHE PATENTSCHRIFT

(45) Veröffentlichungstag der Patentschrift: **21.09.83**

(51) Int. Cl.³: **C 07 C 118/04,** C 07 C 119/042, C 07 C 23/28, C 07 C 17/08

(21) Anmeldenummer: **80105234.1**

(22) Anmeldetag: **03.09.80**

(54) **Verfahren zur Herstellung von 1-Halogenalkylisocyanaten und 1-Alkenylisocyanaten.**

(30) Priorität: **13.09.79 DE 2937006**

(43) Veröffentlichungstag der Anmeldung: **25.03.81 Patentblatt 81/12**

(45) Bekanntmachung des Hinweises auf die Patenterteilung: **21.09.83 Patentblatt 83/38**

(84) Benannte Vertragsstaaten: **BE CH DE FR GB LI NL**

(56) Entgegenhaltungen:
**DE-A-1 807 494**
**DE-A-1 930 329**
**DE-A-2 210 285**
**US-A-3 816 495**

(73) Patentinhaber: **BASF Aktiengesellschaft, *Carl-Bosch-Strasse* 38, D-6700 Ludwigshafen (DE)**

(72) Erfinder: **Koenig, Karl-Heinz, Dr., Pierstrasse 8A, D-6710 *Frankenthal* (DE)**
Erfinder: **Feuerherd, Karl-Heinz, Dr., Berner Weg 34, D-6700 Ludwigshafen (DE)**
Erfinder: **Oeser, Heinz-Guenter, Dr., Friedrich-Burschell-Weg 19, D-6700 Ludwigshafen (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 1-Halogenalkylisocyanaten und 1-Alkenylisocyanaten

Die Erfindung betrifft ein Verfahren zur Herstellung von 1-Halogenalkylisocyanaten und 1-Alkenylisocyanaten durch Umsetzung von 1-Halogenalkylcarbamidsäurehalogeniden oder 1-Halogenalkylisocyanaten mit α-Pinen.

Die wirtschaftliche Synthese von 1-Alkenylisocyanaten ist wegen der grossen Reaktivität dieser Verbindungen schwierig, da 1-Alkenylisocyanate nicht nur thermisch labil, sondern auch säure-, basen- und hydrolyseempfindlich sind. Die bekanntesten Herstellungsmethoden sind der Curtius-Abbau von substituierten Acrylsäureaziden (J. Org. Chem., Band 26 (1961), Seiten 770 bis 779), die Vakuumpyrolyse von Trisvinylisocyanuraten (DE-AS 1 932 811) und die thermische Spaltung von N-tert.-Alkyl-N-(1-alkenyl)-carbamidsäurechloriden (DE-AS 1 922 412).

Es ist aus Liebigs Annalen der Chemie, Band 762 (1972), Seiten 88 bis 92 und Ber. Band 102 (1969), Seiten 2972 bis 2976 bekannt, araliphatische Ketimine mit Phosgen zu einem Gemisch von N-Chlorkarbonylaralkylketiminen und α-Chloralkylisocyanaten umzusetzen. Mehrfach halogensubstituierte 1-Halogenalkylisocyanate sind durch Halogenierung sowohl von unsubstituierten als auch bereits halogensubstituierten Alkylisocyanaten und Carbamidsäurehalogeniden zugänglich (US-PS 3 437 680; DE-OS 1 418 666).

1-Fluorpropylisocyanat wird bei der Tieftemperatur-Fluorierung von Propylisocyanat neben anderen Reaktionsprodukten erhalten (J. Org. Chem. Band 32 (1967), Seiten 1633 bis 1635).

Es ist aus J. Org. Chem., Band 28 (1963), Seiten 1825 bis 1830 bekannt, dass man Chlormethylisocyanat durch Umsetzung von Hydroxymethylisocyanat mit Thionylchlorid erhält. Entsprechend kann auch das 1,2,2,2-Tetrachloräthylisocyanat hergestellt werden.

Es wurde nun gefunden, dass man Gemische von 1-Halogenalkylisocyanaten der Formel

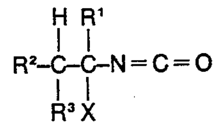

Ia

und Alkenylisocyanaten der Formel

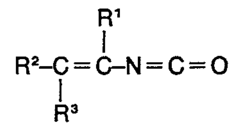

Ib

worin $R^1$, $R^2$ und $R^3$ gleich oder verschieden sein können und jeweils ein Wasserstoffatom oder einen Methylrest bedeuten und X für ein Halogenatom steht, vorteilhaft erhält, wenn man 1-Halogenalkylcarbamidsäurehalogenide der Formel

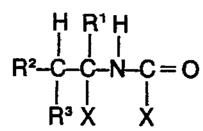

II

oder 1-Halogenalkylisocyanate der Formel

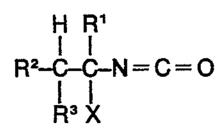

Ia

worin $R^1$, $R^2$, $R^3$ und X die vorgenannte Bedeutung besitzen, mit α-Pinen umsetzt.

Die Umsetzung kann für den Fall der Verwendung von 1-Chloräthylcarbamidsäurechlorid durch die folgenden Formeln wiedergegeben werden:

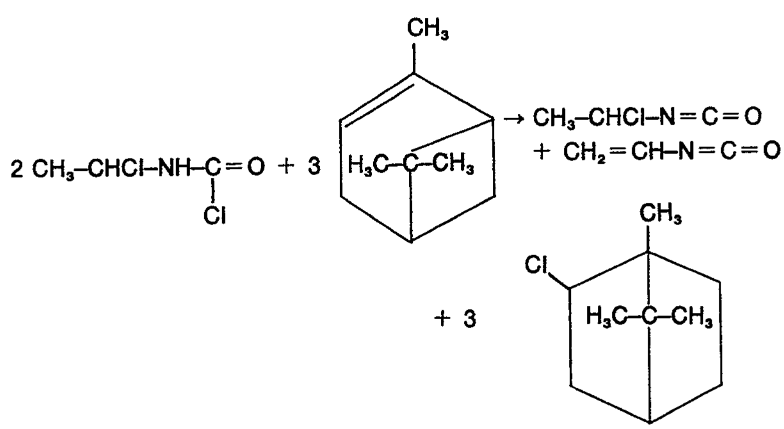

Im Hinblick auf den Stand der Technik liefert das Verfahren nach der Erfindung überraschend auf einfacherem und wirtschaftlicherem Wege 1-Alkenylisocyanate und 1-Halogenalkylisocyanate in guter Ausbeute und Reinheit. Diese vorteilhaften Eigenschaften des erfindungsgemässen Verfahrens waren nicht vorauszusehen.

Denn man konnte nicht erwarten, dass man mit α-Pinen auch Halogenwasserstoff, der nicht von der Carbamidsäurehalogenidgruppe, sondern

von den Atomen des Kohlenstoffgerüsts stammt, unter Ausbildung einer olefinischen Doppelbindung eliminieren kann. Das erfindungsgemässe Verfahren ist um so überraschender, da 1-Alkenylisocyanate bekanntlich ausserordentlich empfindlich gegen Halogenwasserstoff sind und schon bei Raumtemperatur mit diesem harzartige Polymersubstanzen bilden (Recueil, Band 94 (1975), Seite 102) und bei sehr niederen Temperaturen diesen spontan addieren (DE-OS 2 732 284). In Anbetracht des Carbamidsäurechlorid-Isocyanat-Gleichgewichts (Houben-Weyl, Methoden der Organischen Chemie, Band 8, Seite 121) war zu erwarten gewesen, dass entstandenes 1-Alkenylisocyanat mit Halogenwasserstoff aus noch nicht umgesetzten 1-Halogenalkylcarbamidsäurehalogenid zu polymeren Verbindungen abreagieren würde.

Weiterhin ist es überraschend, dass sich nach dem erfindungsgemässen Verfahren neben 1-Alkenylisocyanaten auch 1-Halogenalkylisocyanate herstellen und durch Destillation abtrennen lassen.

Im Falle der Verwendung der Ausgangsstoffe Ia erhält man im wesentlichen die Endstoffe Ib. α-Pinen wird bei dem erfindungsgemässen Verfahren zu Bornylhalogenid umgesetzt. Bevorzugte Ausgangsstoffe II und Ia und demzufolge bevorzugte Endstoffe Ia und Ib sind solche, in deren Formeln die Reste X gleich oder verschieden im Ausgangsstoff II sein können und vorteilhaft ein Bromatom oder insbesondere ein Chloratom bedeuten. Die Ausgangsstoffe II werden auf einfache Weise, z. B. nach dem in der DE-OS 2 741 980 beschriebenen Verfahren, hergestellt. Die nach der Reaktion durch z. B. Destillation aus dem Gemisch abgetrennten Endstoffe Ia können als Ausgangsstoffe erneut wieder für die erfindungsgemässe Umsetzung zur Erzielung eines hohen Anteils an Endstoff Ib verwendet werden.

Geeignete Ausgangsstoffe II sind beispielsweise: 1-Chlorethylcarbamidsäurechlorid, 1-Bromäthylcarbamidsäurebromid, 1-Chlorpropylcarbamidsäurechlorid, 1-Brompropylcarbamidsäurebromid, 1-Chlor-1-methyläthylcarbamidsäurechlorid, 1-Brom-1-methyläthylcarbamidsäurebromid, 2-Chlorbutyl-(2)-carbamidsäurechlorid, 2-Brombutyl-(2)-carbamidsäurebromid, 1-Chlor-2-methylpropylcarbamidsäurebromid, 1-Brom-2-methylpropylcarbamidsäurebromid, 2-Chlor-3-methylbutyl-(2)-carbamidsäurechlorid, 2-Brom-3-methylbutyl-(2)-carbamidsäurebromid.

Geeignete Ausgangsstoffe Ia sind beispielsweise: 1-Chloräthylisocyanat, 1-Bromäthylisocyanat, 1-Chlorpropylisocyanat, 1-Brompropylisocyanat, 1-Chlor-1-methyläthylisocyanat, 1-Brom-1-methyl-äthylisocyanat, 2-Chlorbutyl-(2)-isocyanat, 2-Brombutyl-(2)-isocyanat, 1-Chlor-2-methylpropylisocyanat, 1-Brom-2-methylpropylisocyanat, 2-Chlor-3-methylbutyl-(2)-isocyanat, 2-Brom-3-methylbutyl-(2)-isocyanat.

Die als Endstoffe erhaltenen Gemische von Endstoff Ia und Endstoff Ib enthalten im allgemeinen 0,05 bis 2 Mol Endstoff Ia je Mol Endstoff Ib. Je höher die Reaktionstemperatur und je länger die Reaktionszeit, desto höher ist der Anteil von Endstoff Ib im Endgemisch. Die Umsetzung wird in der Regel bei einer Temperatur von −10 bis 150 °C, im Falle der Herstellung von Gemischen mit mehr als 1,5, insbesondere oberhalb 1,5 bis 2 Mol Endstoff Ia je Mol Endstoff Ib, zweckmässig von 20 bis 50 °C, im Falle der Herstellung von Gemischen mit 0,5 bis 1,5, insbesondere 0,9 bis 1,1 Mol Endstoff Ia je Mol Endstoff Ib, zweckmässig von 70 bis 100°C, im Falle der Herstellung von Gemischen mit weniger als 0,5, insbesondere 0,05 bis unterhalb 0,5 Mol Endstoff Ia je Mol Endstoff Ib, zweckmässig von 100 bis 150 °C drucklos oder unter Druck, kontinuierlich oder diskontinuierlich durchgeführt. Vorteilhaft beginnt man die Umsetzung bei einer Temperatur von −10 bis 30 °C, erhöht langsam die Temperatur und beendet die Reaktion bei den vorgenannten, zweckmässigen Reaktionstemperaturen. Die Reaktionszeit beträgt im allgemeinen 0,5 bis 6 Stunden, vorteilhaft im Falle der Herstellung von Gemischen mit mehr als 1,5, insbesondere oberhalb 1,5 bis 2 Mol Endstoff Ia je Mol Endstoff Ib 4 bis 6 Stunden, im Falle der Herstellung von Gemischen mit 0,5 bis 1,5, insbesondere 0,9 bis 1,1 Mol Endstoff Ia je Mol Endstoff Ib 2 bis 4 Stunden, im Falle der Herstellung von Gemischen mit weniger als 0,5, insbesondere 0,05 bis unterhalb 0,5 Mol Endstoff Ia je Mol Endstoff Ib 0,5 bis 2 Stunden.

Die Menge an α-Pinen wird zweckmässig entsprechend der Menge des abzuspaltenden Halogenwasserstoffs bzw. der gewünschten Menge an Endstoff Ib im Endgemisch gewählt. So verwendet man vorteilhaft 2 bis 20 Mol α-Pinen je Mol Endstoff Ib, bevorzugt für die Herstellung von Gemischen mit mehr als 1,5, insbesondere oberhalb 1,5 bis 2 Mol Endstoff Ia je Mol Endstoff Ib 9 bis 20 Mol α-Pinen je Mol Endstoff Ib, für die Herstellung von Gemischen mit 0,5 bis 1,5, insbesondere 0,9 bis 1,1 Mol Endstoff Ia je Mol Endstoff Ib 4 bis 9 Mol α-Pinen je Mol Endstoff Ib, für die Herstellung von Gemischen mit weniger als 0,5, insbesondere 0,05 bis unterhalb 0,5 Mol Endstoff Ia je Mol Endstoff Ib 3 bis 4 Mol α-Pinen je Mol Endstoff Ib.

Die Reaktion kann wie folgt durchgeführt werden: Ein Gemisch von Ausgangsstoff II oder Ia und α-Pinen wird bei der Reaktionstemperatur während vorgenannter Reaktionszeit umgesetzt. Aus dem Reaktionsgemisch werden dann die Endstoffe Ia und Ib, zweckmässig direkt nach der Reaktion durch Erhöhung der Temperatur und fraktionierte Destillation, abgetrennt. Anstelle der reinen 1-Halogenalkylisocyanate Ia oder 1-Halogenalkylcarbamidsäurehalogenide II lassen sich auch die Reaktionsgemische aus der Herstellung dieser Ausgangsstoffe, z. B. die rohen Halogenierungsgemische verwenden, die bei der Halogenierung von Carbamidsäurehalogeniden anfallen.

Die erfindungsgemäss herstellbaren 1-Alkenylisocyanate Ib und 1-Halogenalkylisocyanate Ia sind wertvolle Ausgangsstoffe für die Herstellung von Schädlingsbekämpfungsmitteln, Farbstoffen, Pharmazeutika, Textilhydrophobierungsmitteln, Waschmitteln, Kunststoffen, Bleichmitteln und Klebstoffen, da sie neben einer reaktiven Isocyanatgruppe noch eine aktivierte Doppelbindung bzw. ein aktiviertes α-C-Atom besitzen. Zudem sind 1-Alkenylisocyanate wichtige Monomere, die zu Ketten- und Leiterpolymeren, wie z.B. strahlungshärtenden Lackharzen, umgesetzt werden können (C.A. 51, (1957), 18694 b–e; J. Polymer Sci. 35 (1959), Seiten 215 bis 218, J. of Coatings Techn. 49 (1977), Heft 632, Seiten 82 bis 86). Sie können Urethanen, z.B. für die Verwendung als Schaumstoffe oder hochmolekulare Überzüge mit hoher Flexibilität, oder Harnstoffen umgesetzt werden. Bezüglich der Verwendung wird auf vorgenannte Veröffentlichungen und Ullmanns Encyklopädie der technischen Chemie, Band 9, Seiten, 11, 12, 404 sowie Band 17, Seite 204 (3. Auflage), hingewiesen.

Die in den folgenden Beispielen aufgeführten Teile bedeuten Gewichtsteile.

Beispiel 1

Ein Gemisch aus 160 Teilen 1-Chloräthylcarbamidsäurechlorid und 537 Teilen α-Pinen wird während 2 Stunden unter Rühren von 24 auf 105 °C erwärmt und das Reaktionsgemisch fraktioniert destilliert. Man erhält insgesamt 27,6 Teile (23% der Theorie) 1-Chloräthylisocyanat vom Kp 92 °C (1013 mbar) und 34,6 Teile Vinylisocyanat vom Kp 38,5 °C (1013 mbar).

Beispiel 2

Ein Gemisch aus 231 Teilen 1-Bromäthylcarbamidsäurebromid und 475 Teilen α-Pinen wird während 1,5 Stunden unter Rühren von 23 auf 120 °C erwärmt und das Reaktionsgemisch fraktioniert destilliert. Man erhält insgesamt 14,6 Teile (10% der Theorie) 1-Bromäthylisocyanat und 61,5 Teile (89% der Theorie) Vinylisocyanat vom Kp 38,5 °C (1013 mbar).

**Patentanspruch**

Verfahren zur Herstellung der Gemische von 1-Halogenalkylisocyanaten der Formel

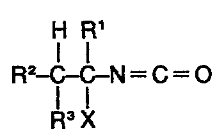

Ia

und 1-Alkenylisocyanaten der Formel

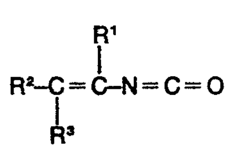

Ib

worin $R^1$, $R^2$ und $R^3$ gleich oder verschieden sein können und jeweils ein Wasserstoffatom oder einen Methylrest bedeuten und X für ein Halogenatom steht, dadurch gekennzeichnet, dass man 1-Halogenalkylcarbamidsäurehalogenide der Formel

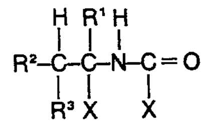

II

oder 1-Halogenalkylisocyanate der Formel

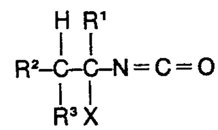

Ia

worin $R^1$, $R^2$, $R^3$ und X die vorgenannte Bedeutung besitzen, mit α-Pinen umsetzt.

**Claim**

A process for the preparation of mixtures of 1-haloalkyl isocyanates of the formula

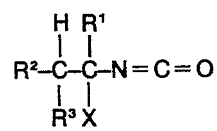

Ia

and 1-alkenyl isocyanates of the formula

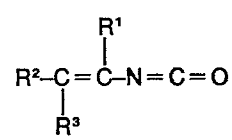

Ib

where $R^1$, $R^2$ and $R^3$ may be identical or different and each is hydrogen or methyl, and X is halogen, wherein a 1-haloalkylcarbamic acid halide of the formula

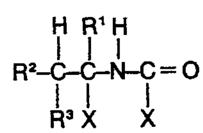

II

or a 1-haloalkyl isocyanate of the formula

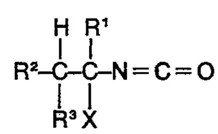

Ia

where $R^1$, $R^2$, $R^3$ and X have the above meanings, is reacted with α-pinene.

**Revendication**

Procédé pour la préparation des mélanges d'isocyanates de 1-halogénalcoyles de formule

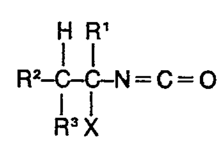

Ia

et d'isocyanates de 1-alcényles de formule

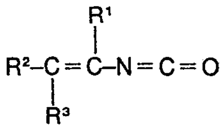

Ib

dans lesquelles $R^1$, $R^2$ et $R^3$ peuvent être semblables ou différents et représentent chacun un atome d'hydrogène ou un radical méthyle et X est mis pour un atome d'halogène, caractérisé en ce qu'on fait réagir des halogénures d'acides 1-halogénalcoylcarbamiques de formule

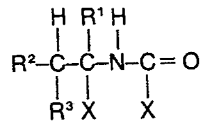

II

ou des isocyanates de 1-halogénalcoyles de formule

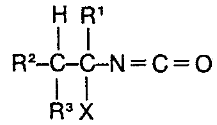

Ia

dans lesquelles $R^1$, $R^2$, $R^3$ et X ont les significations données ci-dessus, avec l'α-pinène.